# EUROPEAN PATENT APPLICATION

(11) **EP 4 074 341 A1**
(43) Date of publication of application: **19.10.2022**
(21) Application number: 20899407.9
(22) Date of filing: 11.12.2020
(51) Int. Cl.: A61K 45/00, A61K 31/155, A61P 9/10

(54) **USE OF NITRIC OXIDE SYNTHASE PATHWAY INHIBITOR IN PREPARATION OF MEDICINE**

(30) Priority: 12.12.2019 CN 201911274663
(71) Applicant: Zhujiang Hospital of Southern Medical University, Guangzhou, Guangdong 510282 (CN)
(72) Inventor: ZHOU, Hongwei, Guangzhou, Guangdong 510282 (CN); XU, Kaiyu, Guangzhou, Guangdong 510282 (CN); GAO, Xuxuan, Guangzhou, Guangdong 510282 (CN); YIN, Jia, Guangzhou, Guangdong 510282 (CN)
(74) Representative: Böhm, Brigitte
(86) International application number: PCT/CN2020/135595
(87) International publication number: WO 2021/115412

(57) **Abstract**

The present application relates to a use of a nitric oxide synthase pathway inhibitor in preparation of a medicine, wherein the medicine is used for preventing, relieving and/or treating gastrointestinal ischemia-reperfusion related distal injury in a subject. The present application further relates to a use of nitric oxide synthase for screening a medicine, wherein the medicine is used for preventing, relieving and/or treating gastrointestinal ischemia-reperfusion related distal injury in a subject. The present application further relates to a pharmaceutical composition containing the nitric oxide synthase pathway inhibitor and a method for preventing, relieving and/or treating gastrointestinal ischemia-reperfusion related distal injury in a subject.

## Description

### TECHNICAL FIELD

The present application relates to the field of biomedicine, and in particular, relates to use of a nitric oxide synthase pathway inhibitor in preparation of a drug.

### BACKGROUND ART

Cerebral stroke is one of the most widespread diseases in the world today, and its incidence rate is increasing year by year. It decreases the quality of life of cerebral stroke patients, and even leads to their deaths in severe cases. Across the world, the annual morbidity and mortality of cerebral ischemic stroke increased by 37% and 21% respectively from 1990 to 2010.

At present, the focus of treatment for cerebral stroke patients is intravenous thrombolysis or endovascular treatment, for example, intravenous injection of a recombinant tissue-type plasminogen activator (r-tPA), which is still considered as the most important therapeutic approach. In addition, depending on the specific disease conditions of each patient, there will be an antiplatelet therapy, an anticoagulant, a neuroprotective agent, and other symptomatic treatments, comprising the treatment and prevention of hyperglycemia, hypertension, and acute stroke complications. However, in view of the increasingly severe form of stroke attacks, there is an urgent need of further developing a new therapeutic method, target, and drug.

### SUMMARY OF THE INVENTION

The present application provides a use of a nitric oxide synthase pathway inhibitor in preparation of a drug for preventing, alleviating and/or treating a distal injury associated with gastrointestinal ischemia-reperfusion in a subject.

In some embodiments, said nitric oxide synthase pathway inhibitor inhibits the activity of nitric oxide synthase.

In some embodiments, said nitric oxide synthase comprises an inducible nitric oxide synthase (iNOS).

In some embodiments, said nitric oxide synthase pathway inhibitor comprises an amino acid inhibitor and/or a non-amino acid inhibitor.

In some embodiments, said nitric oxide synthase pathway inhibitor comprises said amino acid inhibitor, which comprises aminoguanidine (AG), 1400W, L-NIL and/or isothiourea.

In some embodiments, said nitric oxide synthase pathway inhibitor comprises said non-amino acid inhibitor, which comprises a glucocorticoid, a flavonoid, 2-amino-4-methylpyridine and/or aminopiperidine.

In some embodiments, said drug is formulated such that said nitric oxide synthase pathway inhibitor exerts an effect locally in a gastrointestinal tract.

In some embodiments, said drug is formulated such that at or after about 48 hours after administration, said nitric oxide synthase pathway inhibitor is still locally present in the gastrointestinal tract in an effective amount for preventing, alleviating and/or treating said distal injury associated with gastrointestinal ischemia-reperfusion.

In some embodiments, said drug is formulated such that at or after about 1 hour after administration, up to 50% of said nitric oxide synthase pathway inhibitor in said drug is absorbed by said subject to enter a blood circulation system.

In some embodiments, the concentration of said nitric oxide synthase pathway inhibitor in said drug has a concentration of about 0. 0001% (w/w) to about 90% (w/w).

In some embodiments, said subject has experienced, is experiencing or is at risk of experiencing a surgical operation, disease or condition associated with said gastrointestinal ischemia-reperfusion.

In some embodiments, said disease or condition associated with said gastrointestinal ischemia-reperfusion comprises a cerebral stroke, a trauma, a shock, septicemia, acute pancreatitis or an inflammatory bowel disease.

In some embodiments, said disease associated with said gastrointestinal ischemia-reperfusion comprises a cerebral ischemic stroke.

In some embodiments, said subject has experienced, is experiencing or is at risk of experiencing said gastrointestinal ischemia-reperfusion.

In some embodiments, said distal injury associated with said gastrointestinal ischemia-reperfusion comprises said cerebral ischemic stroke.

In some embodiments, said drug is formulated to adapt to oral administration.

In some embodiments, said nitric oxide synthase pathway inhibitor is not substantially decomposed and/or inactivated by digestive juices.

The present application further provides a use of a nitric oxide synthase for screening a drug for preventing, alleviating and/or treating a distal injury associated with gastrointestinal ischemia-reperfusion in a subject.

In some embodiments, said drug inhibits the expression and/or activity of said nitric oxide synthase.

In some embodiments, said nitric oxide synthase comprises an inducible nitric oxide synthase (iNOS).

The present application further provides a pharmaceutical composition, comprising said nitric oxide synthase pathway inhibitor as defined in the present application and optionally a pharmaceutically acceptable carrier.

The present application further provides a use of a nitric oxide synthase pathway inhibitor in preparation of a health care product for preventing, alleviating and/or treating a distal injury associated with gastrointestinal ischemia-reperfusion in a subject.

The present application further provides a use of a nitric oxide synthase pathway inhibitor in preparation of a biological product for preventing, alleviating and/or treating a distal injury associated with gastrointestinal ischemia-reperfusion in a subject.

The present application further provides a method for preventing, alleviating and/or treating a distal injury associated with gastrointestinal ischemia-reperfusion in a subject, said method comprising administering to said subject said nitric oxide synthase pathway inhibitor as defined in the present application.

In some embodiments, said administering comprises gastrointestinal administration.

In some embodiments, said nitric oxide synthase pathway inhibitor exerts an effect locally in a gastrointestinal tract.

In some embodiments, at or after about 48 hours after administration, said nitric oxide synthase pathway inhibitor is still locally present in the gastrointestinal tract in an effective amount for preventing, alleviating and/or treating said distal injury associated with gastrointestinal ischemia-reperfusion.

In some embodiments, at or after about 1 hour after administration, up to 50% of said nitric oxide synthase pathway inhibitor is absorbed by said subject to enter a blood circulation system.

In some embodiments, an administration dose of said nitric oxide synthase pathway inhibitor is about 0.01 to 1000 mg/kg body weight.

The present application further provides a method for preventing a distal injury associated with gastrointestinal ischemia-reperfusion in a subject, said method comprising:
a) monitoring a gastrointestinal condition of said subject; and
b) when said monitoring shows that said subject is at risk of experiencing said gastrointestinal ischemia-reperfusion, or during or after experiencing of said gastrointestinal ischemia-reperfusion, administering to said subject a nitric oxide synthase pathway inhibitor.

In some embodiments, said administering comprises gastrointestinal administration.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:
FIG. 1 shows changes in cecal blood flow in mice observed by a laser speckle imaging system (RWD RFLSI Pro) according to the present application;
FIG. 2 shows the statistical results of ROI proportion of the cecal blood flow in mice observed by the laser speckle imaging system (RWD RFLSI Pro) according to the present application;
FIGs.3A-3C show changes in the expression levels of *Nos2, Nox1*, and *Duox2* genes in intestinal (colonic) tissues after gastrointestinal ischemia-reperfusion induced by cerebral ischemic stroke according to the present application;
FIG. 4 shows changes in nitrate concentration at a cecal mucus layer after gastrointestinal ischemia-reperfusion induced by cerebral ischemic stroke according to the present application;
FIGs. 5A-5C show the effect of aminoguanidine (AG) intervention on the expression levels of *Nos2*, Nox1 and *Duox2* genes in colonic tissues after gastrointestinal ischemia-reperfusion induced by cerebral ischemic stroke according to the present application;
FIG. 6 shows the effect of aminoguanidine (AG) intervention on nitrate concentration at the cecal mucus layer after gastrointestinal ischemia-reperfusion induced by cerebral ischemic stroke according to the present application;
FIGs. 7A-7J show the effect of aminoguanidine (AG) intervention on the expression levels of intestinal barrier-related factors and proinflammatory cytokines in the intestinal tissues after gastrointestinal ischemia-reperfusion induced by cerebral ischemic stroke according to the present application;
FIG. 8 shows the effect of aminoguanidine (AG) intervention on brain injury after gastrointestinal ischemia-reperfusion in mice according to the present application;
FIG. 9 shows the effect of aminoguanidine (AG) intervention on the results of mouse modified neurological severity score according to the present application;
FIGs. 10A-10B show the effect of aminoguanidine (AG) intervention, indicated by Nissl staining, on cerebral neuronal death in mice according to the present application;
FIGs. 11A-11E show the effect of aminoguanidine (AG) intervention on the levels of intestinal barrier markers and inflammatory factors in serum after the gastrointestinal ischemia-reperfusion induced by cerebral ischemic stroke according to the present application; and
FIGs. 12A-12B show the effect of aminoguanidine (AG) intervention at different times or doses on the cerebral ischemic stroke after intestinal ischemia-reperfusion according to the present application.

### DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

As used herein, the term "nitric oxide synthase", usually abbreviated as NOS, generally refers to the collective name of a group of enzymes, which can usually synthesize nitric oxide from nitrogen atoms in arginine in an aerobic environment. In general, the activity of nitric oxide synthase still requires the participation of a cofactor, which may comprise nicotinamide adenine dinucleotide phosphate (NADPH), flavin adenine dinucleotide (FAD), flavin mononucleotide (FMN), proheme, and tetrahydrobiopterin (BH4), etc. The nitric oxide synthase usually may comprise the following three types: I-type neural nitric oxide synthase (nNOS or NOS1), II-type inducible nitric oxide synthase (iNOS or NOS2), and III-type endothelial nitric oxide synthase (eNOS or NOS3). The I- and II-type enzymes are native enzymes (cNOSs), also known as constitutive NOSs (cNOSs), which are enzymes that basically exist in many normal tissues.

As used herein, the term "inducible nitric oxide synthase (iNOS)" generally refers to nitric oxide synthase that is usually expressed only after induction under certain pathological or physiological conditions. Its activity usually does not depend on calcium ions and calmodulin, and large amounts of nitric oxide can be synthesized thereby. Factors that induce iNOS may comprise hemes, cytokines, aerobic stresses, IFN-γ, TNF-α, IL-1α, etc. The inducible nitric oxide synthase (iNOS) may comprise human inducible nitric oxide synthase (iNOS). The human inducible nitric oxide synthase (iNOS) gene is located at position 17cen-q11.2 of the 17th pair of autosomes and is about 37 kb in length. Its transcription is controlled by cytokines, lipopolysaccharide (LPS), and other inflammatory mediators. The promoter of human iNOS is one of the largest and most complex promoters known so far, and it interacts with a transcriptional regulator to control the expression of iNOS genes. The open reading frame of iNOS is encoded by 27 exons, comprising a translation start site (in exon 2) and a stop site (in exon 27). The TATA box of human iNOS is located 30 bp upstream a transcription start site. About 6% of iNOS transcriptions in macrophages and epithelial cells are initiated at multiple start sites, and some even extend across upstream areas of hundreds of base pairs from the main start site directed by TATA. In this way, iNOS mRNA obtained by alternative splicing is more diverse and shows different stability.

As used herein, the term "nitric oxide synthase pathway" generally refers to an intracellular and/or extracellular signaling pathway that regulates the expression or activity of nitric oxide synthase.

As used herein, the term "ischemia" generally refers to a condition where the blood supply to a tissue or organ is insufficient, leading to hypoxia and nutrient deficiency. In general, ischemia may be caused by a vascular problem, such as vascular embolism and vascular compression. It may also be caused by local ischemia induced by vasoconstriction, thrombosis, or embolism, or caused by accidental trauma and surgical intervention, or caused by diseases of other organs or tissues, such as ischemic diseases of other organs or tissues, for example, the gastrointestinal ischemia caused by cerebral ischemic stroke in the present application.

As used herein, the term "ischemia-reperfusion" generally refers to a process where the blood supply returns to the tissues after ischemia or hypoxia (anoxia or low oxygen). In other words, the ischemia-reperfusion generally refers to a process where the blood flow to an ischemic tissue or organ restores after the cause of ischemia is eliminated/counteracted/compensated/retarded. In some embodiments, the reperfusion to the ischemic tissues may be usually accompanied by any of the following conditions: a microvascular system at a reperfusion site is damaged, for example, fluid filtration and diffusion in tissues increase due to increased permeability of capillaries and arterioles; more reactive oxygen species or free radicals are produced by activated endothelial cells after reperfusion, leading to an inflammatory response; newly returned blood transports white blood cells to a reperfusion area, and the white blood cells release, in response to tissue damage, inflammatory factors such as interleukins and free radicals, and may also bind to the endothelium of small capillaries, blocking these capillaries to result in another ischemia; and on the other hand, the restored blood flow reintroduces oxygen into the tissues, the oxygen may destroy the structures of protein, nucleic acid and plasma membrane of cells in this particular case, such that resulting reactive substances may indirectly act on redox signal transduction to initiate cell apoptosis, and the damage to a cell membrane may further lead to the release of more free radicals. The free radicals may comprise free radicals of nitroxide, such as nitric oxide or the derivatives thereof. The ischemia-reperfusion generally leads to reperfusion injury, such as cerebral infarction, acute myocardial infarction, cerebral no reflow phenomenon after cardiopulmonary resuscitation, stress ulcer, pancreatitis, burns, transplantation of isolated organs, intestinal ischemia, necrotizing enterocolitis, intermittent claudication, acute tubular necrosis, liver failure after shock, and multiple organ failure syndrome. In the case that the site with the reperfusion injury is different from the tissue or organ to which ischemia-reperfusion has occurred, it can be generally referred to as distal reperfusion injury.

As used herein, the term "distal injury" generally refers to a pathophysiological process involving organs or tissues that are different from the local organ or tissue that has undergone ischemia-reperfusion. This process may be usually caused by the exposure of cell products, produced by a part of an ischemia-reperfusion organ or tissue, to other organs via the circulatory system. In some embodiments, the distal injury may comprise a disease, condition, or medical intervention inducing the ischemia-reperfusion, for example, a cerebral stroke, a trauma, a shock, septicemia, acute pancreatitis, an inflammatory bowel disease, or a traumatic brain surgery. In some embodiments, the ischemia-reperfusion may comprise gastrointestinal ischemia-reperfusion. In some embodiments, the distal injury may comprise an injury in cerebral ischemic stroke associated with gastrointestinal ischemia-reperfusion, for example, an injury in cerebral ischemic stroke associated with gastrointestinal ischemia-reperfusion as caused by cerebral ischemic stroke.

As used herein, the term "distal injury associated with gastrointestinal ischemia-reperfusion" generally refers to a pathophysiological process of an organ or tissue other than a part of the gastrointestinal tract where ischemia-reperfusion occurs. This pathophysiological process is associated with the gastrointestinal ischemia-reperfusion, and may, for example, be manifested as a pathophysiological process of an organ or tissue other than a part of the gastrointestinal tract, which occurs during, before or after the gastrointestinal ischemia-reperfusion. For example, when intervention is made with respect to the gastrointestinal ischemia-reperfusion or an effect or characterization associated with the gastrointestinal ischemia-reperfusion, the pathophysiological process of the organ or tissue show corresponding changes, for example, symptom relief or alleviation. For example, in the present application, the symptoms of cerebral ischemic stroke are relieved after the nitric oxide synthase inhibitor is administered based on gastrointestinal ischemia-reperfusion.

As used herein, the term "inhibitor" generally refers to a compound/substance or composition that is capable of completely or partially preventing or reducing the physiological function of one or more specific proteins. Reducing the physiological function of one or more specific proteins may involve a reduction in the activity of the protein itself or in the amount of the protein itself. In some embodiments, the inhibitor may exist as different crystals, amorphous substances, pharmaceutically acceptable salts, hydrates, and solvates.

As used herein, the term "administration" generally refers to the introduction of the inhibitor into the subject's body by any route of introduction or delivery. Any method known to a person skilled in the art for contacting a cell, an organ or a tissue with the inhibitor may be used, comprising but not limited to intra-arterial, intranasal, intra-abdominal, intravenous, intramuscular, subcutaneous transdermal or oral administration. A daily dose may be divided into one, two or more doses of a suitable form for administration at one, two or more times during a certain period of time.

As used herein, the term "effective amount" or "effective dose" generally refers to an amount sufficient to achieve or at least partially achieve the desired effect. The "therapeutically effective amount" or "therapeutically effective dose" of a drug or therapeutic agent generally means any drug dose that promotes the regression of a disease (this is demonstrated by means of reduced severity of disease symptoms, increased frequency and duration of the asymptomatic period of the disease, or prevention of injury or disability caused by the disease) when used alone or in combination with another therapeutic agent. The "prophylactically effective amount" or "prophylactically effective dose" of a drug generally refers to a drug dose that inhibits the development or recurrence of a disease when administered alone or in combination with another therapeutic agent to a subject at risk of disease progression or recurrence. A variety of methods known to a person skilled in the art may be used to evaluate the ability of therapeutic or prophylactic agent to promote disease regression or inhibit disease progression or recurrence, for instance, in a human subject undergoing a clinical trial, in an animal model system for predicting the efficacy on humans, or in an in vitro assay for measuring the activity of an agent.

As used herein, the term "cerebral stroke" generally refers to an acute cerebrovascular disease and is also known as "stroke" or "cerebrovascular accident (CVA)". The cerebral stroke may be a group of diseases, comprising cerebral ischemic stroke and cerebral hemorrhagic stroke, which cause brain tissue damage due to the interruption of blood flow to the brain caused by the sudden rupture of a cerebral blood vessel or vascular obstruction.

As used herein, the term "cerebral ischemic stroke" generally refers to a group of diseases that cause nervous tissue dysfunction in a specific area of the brain or a broad brain tissue area due to its insufficient blood supply. The insufficient blood supply to the brain may be caused by a variety of diseases or disorders, such as sickle cell anemia, vascular compression, ventricular tachycardia, arterial plaque accumulation, thrombosis, severe hypotension, and congenital heart defects. The sickle blood cells are more likely to aggregate than normal blood cells, which would impede blood from flowing to the brain; the vascular compression may cause cerebral ischemia by blocking the artery that carries oxygen into the brain, and the causes of vascular compression comprise for example tumors; the ventricular tachycardia may cause complete cardiac arrest to result in stopped blood flow, and arrhythmia may also lead to the formation of blood clots, resulting in cerebral ischemia; arterial blockage caused by the arterial plaque accumulation may also lead to cerebral ischemia, and in the case of little plaque accumulation, it may also lead to narrowed passages in which thrombus is tend to be formed, resulting in cerebral ischemia; in the case of coagulation disorders, large blood clots may also stop the blood flow to induce cerebral ischemia; a heart attack may also cause cerebral ischemia, since the blood flow may be slowed down after the heart attack and the blood may start to coagulate and stop the blood from flowing to the brain; and there is a correlation between the heart attack and hypotension, and the improper use of a drug and the response to the drug may also lead to excessive hypotension, which generally represent insufficient oxygenation of tissues.

In the present application, the cerebral ischemic stroke may also be achieved by surgically blocking the blood flow in a cerebral artery in mice. The nerve tissue dysfunction may comprise neuronal death. The main symptoms of nerve tissue dysfunction in different areas may comprise: changes in olfaction/gustation/auditory or visual sensation, dysphagia and asthenocoria to light, physical dyskinesia, aphasia, changes in breathing and heart rate, ischemia of other tissues or organs, loss of consciousness, etc.

As used herein, the term "digestive juice" generally refers to a fluid secreted by the digestive system for digesting food. The digestive juice is mainly composed of organic matter, ions, and water. The main functions of digestive juice may comprise: diluting food to achieve the same osmotic pressure as that of plasma in order to facilitate absorption; changing the pH in the digestive cavity to adapt to the needs of digestive enzyme activity; hydrolyzing complex food components to make it easier to absorb; protect the mucosa of the digestive tract and prevent physical and chemical injury by secreting mucus, antibodies and a large amount of fluid. The digestive juice may comprise the followings: saliva, gastric juice, pancreatic juice, bile, small intestinal juice, etc.

As used herein, the term "prevention" generally refers to a prophylactic administration combination for a healthy subject in order to prevent the occurrence of a certain disease or condition. It may also comprise a prophylactic administration combination for a patient in the early stage of an allergic disease to be treated. "Prevention" does not require the possibility of 100% elimination of the occurrence of a disease or condition. In other words, "prevention" generally means reducing the probability or degree of occurrence of a disease or condition in the presence of the administration combination.

As used herein, the term "alleviating" means decreasing, reducing, or delaying a certain condition, disease, disorder, or phenotype. The condition, disease, disorder, or phenotype may comprise subjective perceptions such as pain, dizziness or other physiological disorders of a subject, or medically detectable indications, such as the condition of a lesion detected by medical testing means.

As used herein, the term "treatment" generally refers to a clinical intervention used to change a natural course in a treated individual or cell in a clinical pathological process. It may comprise improving the state of a disease, eliminating a lesion, or improving prognosis.

As used herein, the term "gastrointestinal administration" generally refers to allowing the specific release of a therapeutic agent in an area at or near the gastrointestinal tract. By local instead of systemic release of the therapeutic agent, the bioavailability of a drug may be increased in part of the gastrointestinal tract and/or decreased in the systemic circulation, and a lower systemic drug level may lead to reduced toxicity and reduced immunogenicity (for example, in the case of a biological agent), which on the one hand, results in improved overall safety and fewer adverse side effects, and on the one hand, increases the dose in the part of the gastrointestinal tract to achieve a more efficient and targeted effect. In some cases, the topical administration of a therapeutic agent also provides a new mode of action, for example, combined administration at different local locations.

As used herein, the term "exerts an effect locally in the gastrointestinal tract" generally refers to that a therapeutic agent administered can maintain an effective dose locally in the gastrointestinal tract and change the local condition or phenotype in the gastrointestinal tract. Exerting the effect locally in the gastrointestinal tract does not exclude observing the therapeutic agent in organs or tissues other than the gastrointestinal tract. Generally, the therapeutic agent may have stability in the part of the gastrointestinal tract and/or a tissue penetration capability (the capability to penetrate into the gastrointestinal tissues), and may be substantially distributed in the gastrointestinal tissues. The stability may comprise the stability before and/or after administration, for example, the stability in a delivery device, and the stability of a formulation and/or drug in a gastrointestinal environment after administration (comprising the gastrointestinal environment in a disease state), such as temperature stability, pH stability, oxidation stability. Locally in the gastrointestinal tract may involve a portion or sub-portion of one or more parts of the gastrointestinal tract of a subject.

As used herein, the term "subject" generally refers to a human or non-human animal, comprising but not limited to a cat, a dog, a horse, a pig, a cow, a goat, a rabbit, a mouse, a rat, or a monkey.

As used herein, the term "pharmaceutical composition" generally refers to a mixture, which comprises at least one active ingredient to be administered to a subject to treat a specific disease or condition affecting the individual. It allows the active ingredient to remain in an effective form and comprises no additional component that has unacceptable toxicity to the subject to which the composition is to be administered. This composition may be sterile, or may comprise a pharmaceutically acceptable carrier.

As used herein, the term "pharmaceutically acceptable carrier" generally refers to a pharmaceutically acceptable substance, composition or vehicle involved in carrying or transporting a chemical agent, for example, a buffer, a surfactant, a stabilizer, a preservative, an absorption enhancer for enhancing bioavailability, a liquid or solid filler, a diluent, an excipient, a solvent, an encapsulating material and/or other conventional solubilizer or dispersants.

As used herein, the term "comprise" generally refers to the inclusion of explicitly specified features, but not excluding other elements.

As used herein, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

In one aspect, the present application provides a use of a nitric oxide synthase pathway inhibitor in preparation of a drug for preventing, alleviating and/or treating a distal injury associated with gastrointestinal ischemia-reperfusion in a subject. The present application further provides a use of a nitric oxide synthase pathway inhibitor in preparation of a drug for preventing, alleviating and/or treating a disease or condition associated with the nervous system in a subject. In the present application, the drug is formulated such that the nitric oxide synthase pathway inhibitor exerts an effect locally in the gastrointestinal tract.

In the present application, locally in the gastrointestinal tract may involve parts such as the stomach, small intestine, and large intestine. The stomach may comprise gastric cardia, the fundus of stomach, the body of stomach, and the pylorus of stomach. The small intestine may comprise duodenum, jejunum, and ileum. The large intestine may comprise cecum, colon, and rectum.

In the present application, exerting an effect locally in the gastrointestinal tract may comprise the nitric oxide synthase pathway inhibitor inhibiting the activity of the nitric oxide synthase locally in the gastrointestinal tract or reducing the expression of the nitric oxide synthase locally in the gastrointestinal tract.

For example, exerting an effect locally in the gastrointestinal tract may involve a decrease in the expression level of the *iNOS* gene in the intestinal tissue. The intestinal tissues may comprise colon tissues.

In the present application, exerting the effect locally in the gastrointestinal tract may comprise a decrease in nitrate level in intestinal tissues. The intestinal tissues may comprise cecum tissues.

In the present application, exerting the effect locally in the gastrointestinal tract may comprise a decrease in the expression level of *Nox1 or Duox2* gene in intestinal tissues. The intestinal tissues may comprise colon tissues.

In the present application, exerting the effect locally in the gastrointestinal tract may comprise a change in the expression level of an intestinal barrier related gene in intestinal tissues.

For example, the intestinal barrier related gene may comprise *Tjp1*, *Ocln*, and *Cldn2. Tjp1* is tight junction protein 1, *Ocln* is Occludin, and *Cldn2* is Claudin-2.

For example, exerting the effect locally in the gastrointestinal tract may comprise an increase in the expressions of *Tjp1* and *Ocln* in intestinal tissues.

For example, exerting the effect locally in the gastrointestinal tract may comprise a decrease in the expression of *Cldn2* in intestinal tissues.

In the present application, exerting the effect locally in the gastrointestinal tract may comprise a decrease in the expression level of a proinflammatory cytokine gene in intestinal tissues. The proinflammatory cytokine gene is any one selected from the group consisting of: *Tnf, Il17, Ifng, Il1b, 116, Cxcl2, and Kc*; wherein *Tnf* is a tumor necrosis factor, *Il17* is interleukin 17, *Ifng* is interferon γ, *Il1b* is interleukin 1β, *Il6* is interleukin 6, *Kc* and *Cxcl2* are chemokine members.

For example, the intestinal tissues may comprise jejunum, ileum, cecum or colon.

In the present application, the drug is formulated such that at or after about 48 hours after administration, the nitric oxide synthase pathway inhibitor is still locally present in the gastrointestinal tract in an effective amount for preventing, alleviating and/or treating the distal injury associated with gastrointestinal ischemia-reperfusion.

In the present application, the drug is formulated such that at or after about 1 hour after administration, up to 50% of the nitric oxide synthase pathway inhibitor in the drug is absorbed by the subject to enter a blood circulation system.

In the present application, the concentration of the nitric oxide synthase pathway inhibitor in the drug is from about 0.0001% (w/w) to about 90% (w/w).

For example, the concentration of the nitric oxide synthase pathway inhibitor is from about 0.0005% (w/w) to about 90% (w/w), from about 0.001% (w/w) to about 85% (w/w), from about 0.0015% (w/w) to about 80% (w/w), from about 0.002% (w/w) to about 75% (w/w), from about 0.0025% (w/w) to about 70% (w/w), from about 0.003% (w/w) to about 65% (w/w), from about 0.0035% (w/w) to about 60% (w/w), from about 0.004% (w/w) to about 55% (w/w), from about 0.0045% (w/w) to about 50% (w/w), from about 0.005% (w/w) to about 45% (w/w), from about 0.0055% (w/w) to about 40% (w/w), from about 0.006% (w/w) to about 35% (w/w), from about 0.0065% (w/w) to about 30% (w/w), from about 0.007% (w/w) to about 25% (w/w), from about 0.0075% (w/w) to about 20% (w/w), from about 0.01% (w/w) to about 80% (w/w), from about 0.1% (w/w) to about 70% (w/w), from about 0.5% (w/w) to about 60% (w/w), from about 1% (w/w) to about 50% (w/w), from about 5% (w/w) to about 90% (w/w), about 10% (w/w) to about 80% (w/w), about 20% (w/w) to about 70% (w/w), about 10% (w/w) to about 50% (w/w), from about 20% (w/w) to about 50% (w/w), from about 20% (w/w) to about 40% (w/w), from about 30% (w/w) to about 50% (w/w), and from about 10% (w/w) to about 20% (w/w).

In the present application, the subject has suffered from, is suffering from or is at risk of suffering from a disease or condition associated with the gastrointestinal ischemia-reperfusion.

In the present application, the disease or condition associated with gastrointestinal ischemia-reperfusion may comprise a natural event, a trauma, or one or more surgical operations or other therapeutic interventions for reducing/stopping blood flow in the gastrointestinal tract. The natural event may comprise arterial infarction, venous obstruction, or systemic hypotension that destroys or reduces blood flow to internal organs, and the systemic hypotension may comprise a hemorrhagic shock caused by blood loss, a cardiogenic shock caused by myocardial infarction or heart failure, a neurogenic shock, a renal shock, or an allergic reaction.

For example, the disease and/or condition associated with the gastrointestinal ischemia-reperfusion may comprise a cerebral stroke, a trauma, a shock, septicemia, acute pancreatitis and/or an inflammatory bowel disease.

For example, the disease associated with the gastrointestinal ischemia-reperfusion may comprise a cerebral stroke. The cerebral stroke may be a group of diseases that cause brain tissue damage due to the interruption or reduction of blood flow to the brain caused by the sudden rupture of a cerebral blood vessel or vascular obstruction.

For example, the disease may comprise the cerebral ischemic stroke. The cerebral ischemic stroke may comprise atherosclerotic occlusion of a large artery, cerebral embolism (embolic infarction), non-embolic infarction of a small deep perforating artery (lacunar cerebral infarction), and ischemia in a watershed region due to distal arteriostenosis and decreased cerebral blood flow (hemodynamic stroke).

In the present application, the subject has suffered from the above-mentioned disease or condition associated with the gastrointestinal ischemia-reperfusion. The expression "has suffered" may comprise the fact that the subject has suffered from the above-mentioned disease associated with the gastrointestinal ischemia-reperfusion prior to administration of the drug to the subject.

In the present application, the subject is suffering from the above-mentioned disease associated with the gastrointestinal ischemia-reperfusion. The expression "is suffering" may comprise the fact that the subject suffers from the above-mentioned disease associated with the gastrointestinal ischemia-reperfusion during administration of the drug to the subject.

In the present application, the subject is at risk of suffering from the above-mentioned disease associated with the gastrointestinal ischemia-reperfusion. The expression "at risk of suffering" may comprise the fact that the subject may suffer from the above-mentioned disease associated with the gastrointestinal ischemia-reperfusion after administration of the drug to the subject.

In the present application, the subject has suffered from, is suffering from, or is at risk of suffering from the gastrointestinal ischemia-reperfusion.

For example, the subject has experienced the gastrointestinal ischemia-reperfusion. The expression "has experienced" may comprise the fact that the subject has experienced the gastrointestinal ischemia-reperfusion prior to administration of the drug to the subject.

For example, the subject is experiencing the gastrointestinal ischemia-reperfusion. The expression "is experiencing" may comprise the fact that the subject is experiencing the gastrointestinal ischemia-reperfusion during administration of the drug to the subject.

For example, the subject is at risk of experiencing the gastrointestinal ischemia-reperfusion. The expression "at risk of experiencing" may comprise the fact that the subject has experienced the gastrointestinal ischemia-reperfusion after administration of the drug to the subject.

For example, the distal injury associated with the gastrointestinal ischemia-reperfusion comprises the cerebral ischemic stroke.

In the present application, the drug is formulated to adapt to be orally administered, for example, oral administration.

For example, dosage forms for oral administration may comprise capsules, tablets, pills, granules or syrups.

For example, oral doses may be administered in doses ranging from about 0.01 to 1000 mg/kg, from about 0.01 to 200 mg/kg, from about 0.01 to 180 mg/kg, from about 0.01 to 160 mg/kg, from about 0.01 to 140 mg/kg, from about 0.01 to 120 mg/kg, from about 0.01 to 100 mg/kg, from about 0.1 to 200 mg/kg, from about 0.1 to 150 mg/kg, from about 0.1 to 100 mg/kg, from about 0.1 to 80 mg/kg, from about 1 to 60 mg/kg, from about 0.1 to 40 mg/kg, from about 0.1 to 20 mg/kg, from 25 to 200 mg/kg, from about 50 to 200mg/kg, from about 100 to 200 mg/kg, from about 25 to 50 mg/kg, from about 25 to 100 mg/kg, from about 50 to 100 mg/kg, and from about 50 to 200 mg/kg body weight.

For example, a method for oral administration may comprise administrating one or more times a day, daily, every other day, every week, every two weeks, every month, or every two months.

For example, the time for oral administration is about 1-7 days, about 1-6 days, about 1-5 days, about 1-4 days, about 1-3 days, about 1-2 days, 1-24 hours, about 3-12 hours, about 6-12 hours, about 1-3 hours, about 1-6 hours, about 3-6 hours, about 3-12 hours, about 6-12 hours after stroke.

In the present application, the nitric oxide synthase pathway inhibitor is not substantially decomposed and/or inactivated by digestive juices. The digestive juices may comprise saliva, gastric juice, small intestinal juice, pancreatic juice, and bile.

For example, saliva, with a pH of 6.6 to 7.1, has the main components comprising salivary amylase, lysozyme and a small amount of inorganic substances (such as inorganic salts comprising sodium, potassium, and calcium), etc. The nitric oxide synthase pathway inhibitor is not substantially decomposed and/or inactivated by the saliva.

For example, gastric juice, with a pH of 0.9 to 1.5, has the main components comprising pepsin, gastric acid (i.e., hydrochloric acid), mucus, and inorganic substances such as sodium salts and potassium salt. The nitric oxide synthase pathway inhibitor is not substantially decomposed and/or inactivated by the gastric juice.

For example, pancreatic juice, with a pH of 7.8 to 8.4, has the main components comprising sodium bicarbonate, pancreatic amylase, pancreatic lipase, trypsinogen and chymotrypsinogen, etc.; and the nitric oxide synthase pathway inhibitor is not substantially decomposed and/or inactivated by the pancreatic juice.

For example, bile, with a pH of about 6.8 to 7.4, has the main components comprising bile salts and bile pigments. The nitric oxide synthase pathway inhibitor is not substantially decomposed and/or inactivated by the bile.

For example, small intestinal juice, with a pH of about 7.6, comprises various digestive enzymes such as amylase, maltase, sucrase, lactase, peptidase and lipase. The nitric oxide synthase pathway inhibitor is not substantially decomposed and/or inactivated by the small intestinal juice.

For example, the nitric oxide synthase pathway inhibitor is not substantially decomposed and/or inactivated by the saliva, gastric juice, pancreatic juice, bile, and small intestinal juice.

For example, the nitric oxide synthase pathway inhibitor is not substantially decomposed and/or inactivated when the pH is 6.6-7.1, 0.9-1.5, 7.8-8.4, 6.8-7.4, or 7.6.

The nitric oxide synthase pathway inhibitor being not substantially decomposed and/or inactivated by the digestive juices may comprise that the nitric oxide synthase pathway inhibitor is capable of substantially maintaining its performance in inhibiting the nitric oxide synthase when it is in the digestive juices or after its contact with the same.

In another aspect, the present application further provides a use of a nitric oxide synthase for screening a drug for preventing, alleviating and/or treating a distal injury associated with gastrointestinal ischemia-reperfusion in a subject.

The present application further provides a use of a nitric oxide synthase pathway inhibitor in screening of a drug for preventing, alleviating and/or treating a disease or condition associated with the nervous system in a subject.

In the present application, the screening of the drug may comprise a process for evaluating the biological activity, pharmacological effects and medicinal value of substances that may be used as drugs. The screening of the drug may comprise screening at biochemical and cellular levels. The screening of the drug may further comprise high-throughput screening and virtual drug screening.

In some embodiments, said drug inhibits the expression and/or activity of said nitric oxide synthase.

In some embodiments, the nitric oxide synthase is an inducible nitric oxide synthase (iNOS).

In another aspect, the present application further provides a pharmaceutical composition, which may comprise the nitric oxide synthase pathway inhibitor as defined in the present application and optionally a pharmaceutically acceptable carrier.

For example, the pharmaceutical composition may comprise a health care product.

The health care product, also called a dietary supplement, generally refers to a class of products that share the common characteristics of general foods, can regulate the functions of a human body, and is suitable for consumption by a specific group of people, but is not for the purpose of curing diseases. It comprises tea, wine, bee products, drinks, soup, fresh juice, medicated diet, etc.

For example, the pharmaceutical composition may comprise a biological product.

The biological product generally refers to a product prepared from biological materials such as a microorganism, a cell and various animal and human-derived tissues and liquids obtained by using ordinary technologies or biotechnologies such as genetic engineering, cell engineering, protein engineering, and fermentation engineering, for the purpose of preventing, treating and diagnosing a human disease. It comprises a bacterial vaccine, a vaccine, a toxin, a toxoid, a blood product, an immunoglobulin, an antigen, an allergen, a cytokine, a hormone, an enzyme, a fermentation product, a monoclonal antibody, a DNA recombinant product, etc.

The nitric oxide synthase pathway inhibitor may comprise an amino acid inhibitor and/or a non-amino acid inhibitor. The amino acid inhibitor may comprise aminoguanidine (AG), 1400W, L-NIL and/or isothiourea; and the non-amino acid inhibitor may comprise a glucocorticoid, a flavonoid, 2-amino-4-methylpyridine and/or aminopiperidine. The pharmaceutically acceptable carrier may comprise a pharmaceutically acceptable substance, composition or vehicle, such as a buffer, a surfactant, a stabilizer, a preservative, an absorption enhancer for enhancing bioavailability, a liquid or solid filler, a diluent, a excipient, a solvent, a encapsulating material and/or other conventional solubilizers or dispersant, which are involved in carrying or transporting a chemical agent.

In the present application, the pharmaceutical composition may comprise any one or any combination of the following components: gum arabic, alginate, alginic acid, aluminum acetate, benzyl alcohol, butyl-p-hydroxybenzoate, butylated hydroxytoluene, antioxidant, citric acid, calcium carbonate, candelilla wax, croscarmellose sodium, candy sugar, colloidal silicon dioxide, cellulose, carnauba wax, corn starch, calcium carboxymethyl cellulose, calcium stearate, calcium disodium EDTA, copovidone, hydrogenated castor oil, dehydrated calcium hydrophosphate, cetylpyridinium chloride, cysteine HC1, crospovidone, calcium hydrophosphate, disodium hydrophosphate, polydimethylsiloxane, sodium erythrosine, ethyl cellulose, gelatin, glyceryl monooleate, glycerin, glycine, glyceryl monostearate, glyceryl behenate, hydroxypropyl cellulose, hydroxypropyl methylcellulose, hypromellose, HPMC phthalate, iron oxide, iron oxide yellow, lactose (aqueous or anhydrous or monohydrate or spray-dried), magnesium stearate, microcrystalline cellulose, mannitol, methyl cellulose, magnesium carbonate, mineral oil, methacrylic acid copolymer, magnesium oxide, methyl parahydroxybenzoate, PEG, polysorbate 80, propylene glycol, polyepoxy ethane, propyl parahydroxybenzoate, poloxamer 407 or 188, potassium bicarbonate, potassium sorbate, starch, phosphoric acid, polyoxyethylene 40 stearate, sodium starch glycolate, pregelatinized starch, crosslinked carboxymethyl cellulose, sodium lauryl sulfate, silicon dioxide, sodium benzoate, stearic acid, syrup for medicinal candy, granulating agent, sorbic acid, sodium carbonate, sodium saccharin, sodium alginate, silica gel, sorbitol monooleate, sodium stearyl fumarate, sodium chloride, sodium metabisulfite, dehydrated sodium citrate, sodium carboxymethyl cellulose, succinic acid, sodium propionate, titanium dioxide, talc, glycerin triacetate, or triethyl citrate.

In the present application, the pharmaceutical composition may be in a solid form, for example, a capsule, tablet, pill, granule, sachet or lozenge; or may be in a liquid form, for example, a solution, suspension, emulsion or syrup.

In another aspect, the present application further provides a method for preventing, alleviating and/or treating a distal injury associated with gastrointestinal ischemia-reperfusion in a subject, the method comprising administering to the subject the nitric oxide synthase pathway inhibitor as defined in the present application.

The present application further provides a method for preventing, alleviating and/or treating a disease or condition associated with the nervous system in a subject. The method comprises administering to the subject the nitric oxide synthase pathway inhibitor as defined in the present application. For example, the administering comprises gastrointestinal administration. The gastrointestinal administration comprises specifically releasing the nitric oxide synthase pathway inhibitor at or in the proximity of a part of the gastrointestinal tract.

For example, the nitric oxide synthase pathway inhibitor is delivered to the gastrointestinal tract by means of a mechanical device.

For example, the nitric oxide synthase pathway inhibitor is delivered to the gastrointestinal tract by means of an endoscope and a spray catheter.

For example, the nitric oxide synthase pathway inhibitor is allowed to be released in the gastrointestinal tract by oral administration of a sustained-release agent or a disintegrating agent.

For example, the sustained-release agent or disintegrating agent is capable of initiating release or disintegration depending on the pH of the gastrointestinal tract.

For example, the nitric oxide synthase pathway inhibitor is allowed to be released in the gastrointestinal tract by means of an enteric-coated capsule or a liposome microcapsule.

For example, the nitric oxide synthase pathway inhibitor is intragastrically delivered to the gastrointestinal tract.

For example, the time for gastrointestinal administration is about 1-7 days, about 1-6 days, about 1-5 days, about 1-4 days, about 1-3 days, about 1-2 days, 1-24 hours, about 3-12 hours, about 6-12 hours, about 1-3 hours, about 1-6 hours, about 3-6 hours, about 3-12 hours, about 6-12 hours after stroke.

For example, the nitric oxide synthase pathway inhibitor exerts an effect locally in the gastrointestinal tract.

For example, at or after about 48 hours after administration, the nitric oxide synthase pathway inhibitor is still locally present in the gastrointestinal tract in an effective amount for preventing, alleviating and/or treating the distal injury induced by the gastrointestinal ischemia-reperfusion.

For example, at or after about 1 hour after administration, up to 50% of the nitric oxide synthase pathway inhibitor is absorbed by the subject to enter a blood circulation system.

For example, the administration dose of the nitric oxide synthase pathway inhibitor is from about 0.01 to 1000 mg/kg, from about 0.01 to 200 mg/kg, from about 0.01 to 180 mg/kg, from about 0.01 to 160 mg/kg, from about 0.01 to 140 mg/kg, from about 0.01 to 120 mg/kg, from about 0.01 to 100 mg/kg, from about 0.1 to 200 mg/kg, from about 0.1 to 150 mg/kg, from about 0.1 to 100 mg/kg, from about 0.1 to 80 mg/kg, from about 1 to 60 mg/kg, from about 0.1 to 40 mg/kg, from about 0.1 to 20mg/kg, from 25 to 200 mg/kg, from about 50 to 200 mg/kg, from about 100 to 200 mg/kg, from about 25 to 50 mg/kg, from about 25 to 100 mg/kg, from about 50 to 100 mg/kg, and from about 50 to 200 mg/kg body weight.

In another aspect, the present application further provides a method for preventing a distal injury associated with gastrointestinal ischemia-reperfusion in a subject, the method comprising: (1) monitoring a gastrointestinal condition of the subject; and (2) when or after the monitoring shows that the subject is experiencing the gastrointestinal ischemia-reperfusion, administering to the subject the nitric oxide synthase pathway inhibitor.

For example, the method further comprises, prior to step (1), monitoring a disease or condition associated with the gastrointestinal ischemia-reperfusion.

For example, detecting the gastrointestinal condition of the subject comprises clinical physical examination, clinical testing, and/or clinical examination to evaluate the gastrointestinal condition of the subject.

For example, when it is monitored that a patient has experienced, is experiencing, or is at risk of experiencing a disease or condition associated with gastrointestinal ischemia-reperfusion, clinical physical examination, inspection, and examination are performed to evaluate the gastrointestinal condition of the subject.

For example, the clinical physical examination may comprise observation of symptoms such as appetite, dysphagia, abdominal pain, nausea, vomiting, hematemesis, hematochezia, stool characteristics, abdominal pain and distension during defecation, and changes in defecation habits, and physical examination of the abdominal region, for a patient.

For example, the clinical testing comprises three major routine examinations etc. For example, the clinical testing may comprise blood routine examination, urine routine examination, and stool routine examination.

For example, the clinical examination may comprise abdominal X-ray, ultrasound, CT, endoscopy, ERCP, and PTC examination, etc.

For example, monitoring the gastrointestinal condition of the subject may comprise observing changes in blood flow in a part of the gastrointestinal site.

For example, the administering comprises gastrointestinal administration.

For example, the sustained-release agent or disintegrating agent is capable of initiating release or disintegration depending on the pH of the gastrointestinal tract.

For example, the nitric oxide synthase pathway inhibitor is allowed to be released in the gastrointestinal tract by means of an enteric-coated capsule or a liposome microcapsule.

For example, the nitric oxide synthase pathway inhibitor is intragastrically delivered to the gastrointestinal tract.

In the present application, the nitric oxide synthase may comprise any one of the following: I neural nitric oxide synthase (nNOS or NOS1), II inducible nitric oxide synthase (iNOS or NOS2), III endothelial nitric oxide synthase (eNOS or NOS3).

For example, the nitric oxide synthase may comprise inducible nitric oxide synthase (iNOS or NOS2).

In the present application, the nitric oxide synthase pathway inhibitor inhibits the expression and/or activity of nitric oxide synthase.

For example, the nitric oxide synthase pathway inhibitor inhibits the expression and/or activity of inducible nitric oxide synthase (iNOS).

For example, the nitric oxide synthase pathway inhibitor inhibits the expression and/or activity of inducible nitric oxide synthase (iNOS) in the gastrointestinal tract.

For example, the nitric oxide synthase pathway inhibitor decreases the expression of inducible nitric oxide synthase (iNOS) in the gastrointestinal tract.

For example, the nitric oxide synthase pathway inhibitor decreases the nitrate concentration in the gastrointestinal tract.

For example, the nitric oxide synthase pathway inhibitor may comprise an amino acid inhibitor.

For example, the nitric oxide synthase pathway inhibitor may comprise a non-amino acid inhibitor.

For example, the amino acid inhibitor may comprise aminoguanidine (AG), 1400W, L-NIL and/or isothiourea, etc. The structural formula of 1400W is as follows:

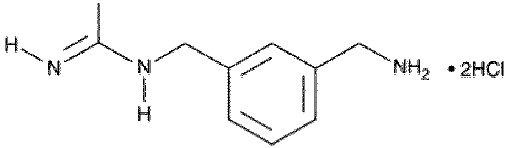

The structural formula of L-NIL is as follows:

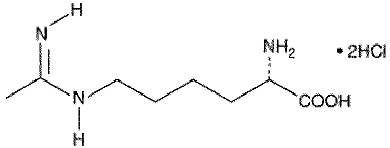

For example, the non-amino acid inhibitor may comprise a glucocorticoid, a flavonoid, 2-amino-4-methylpyridine and/or aminopiperidine, etc.

In the present application, the distal injury associated with gastrointestinal ischemia-reperfusion may comprise a pathological or physiological process of any organ or tissue other than the gastrointestinal tract.

For example, the pathological or physiological process of any organ or tissue other than the gastrointestinal tract may occur concurrently with the gastrointestinal ischemia-reperfusion.

For example, occurring concurrently may refer to that the difference in occurrence time between the pathological or physiological process of any organ or tissue other than the gastrointestinal tract and the gastrointestinal ischemia-reperfusion is within 10 minutes, for example, within 5 minutes, for example, wthin 3 minutes, and for example, within 1 minute.

For example, the pathological or physiological process of any organ or tissue other than the gastrointestinal tract may occur before the gastrointestinal ischemia-reperfusion.

For example, the pathological or physiological process of any organ or tissue other than the gastrointestinal tract may occur within 24 hours, for example within 22 hours, for example within 20 hours, for example within 18 hours, for example within 16 hours, for example within 14 hours, for example within 12 hours, for example within 10 hours, for example within 8 hours, for example within, 6 hours, for example within 5 hours, for example within 4.5 hours, for example within 4 hours, for example within 3.5 hours, for example within 3 hours, for example within 2.5 hours, for example within 2 hours, for example within 1.5 hours, for example within 1 hours, for example within 0.5 hour, before the gastrointestinal ischemia-reperfusion. For example, the pathological or physiological process of any organ or tissue other than the gastrointestinal tract may occur after the gastrointestinal ischemia-reperfusion.

For example, the pathological or physiological process of any organ or tissue other than the gastrointestinal tract may occur within 30 days, for example within 28 days, for example within 26 days, for example within 24 days, for example within 22 days, for example within 20 days, for example within 18 days, for example within 16 days, for example within 14 days, for example within 12 days, for example within 10 days, for example within 8 days, for example within 1 day, for example within 6 days, for example within 5 days, for example within 4 days, for example within 3 days, for example within 2 days, for example within 1 days, for example within 12 hours, for example within 9 hours, for example, within 6 hours, for example within 3 hours, for example within 1 hour, and for example within 0.5 hour, after the gastrointestinal ischemia-reperfusion.

For example, the pathological or physiological process of any organ or tissue other than the gastrointestinal tract may be manifested in that the pathophysiological process of the organ or tissue is alleviated or relieved when intervention is made with respect to the gastrointestinal ischemia-reperfusion or an effect or characterization associated with the gastrointestinal ischemia-reperfusion.

For example, the effect or manifestation associated with intestinal ischemia-reperfusion may comprise changes in the expression of *Nos2, Nox1*, and *Duox2* in gastrointestinal tissues, changes in nitrate level in gastrointestinal tissues, changes in intestinal barriers, and changes in proinflammatory factors in gastrointestinal tissues, wherein *Nos2* encodes inducible nitric oxide synthase, *Nox1* encodes NADPH oxidase 1, *Duox2* encodes dioxygenase 2, and NADPH oxidase 1 and dioxygenase 2 are in connection with the production of reactive oxygen.

For example, the changes in the expression of *iNOS, Nox1*, and *Duox2* in the gastrointestinal tissue may comprise increased expressions of *iNOS, Nox1*, and *Duox2* in the gastrointestinal tissues; the changes in the nitrate level in the gastrointestinal tissues may comprise an increase in the nitrate level in the gastrointestinal tissues; the changes in the intestinal barrier may comprise intestinal barrier injury; and the changes in the proinflammatory factors in the gastrointestinal tissues comprise increased proinflammatory factors in the gastrointestinal tissues.

For example, the intestinal barrier injury may comprise decreased expression levels of Tjp1 and Ocln genes, and an increased expression level of a Cldn2 gene; and the inflammatory factors may comprise Tnf, Il17, Ifng, Il1b, Cxcl2, Kc, and/or Il6, wherein Ocln and Cldn2 are intestinal barrier related genes, Ocln is Occludin, Cldn2 is Claudin-2, Tnf is a tumor necrosis factor, Il17 is interleukin 17, Ifng is interferon γ, Il1b is interleukin 1β, Il6 is interleukin 6, and Kc and Cxcl2 are chemokines.

For example, the changes in the expressions of *iNOS, Nox1*, and *Duox2* in the gastrointestinal tissues may comprise successive increase and decrease in the expressions of *iNOS, Nox1*, and *Duox2* in the gastrointestinal tissues, and the changes in the nitrate level in the gastrointestinal tissues may comprise successive increase and decrease in the nitrate level in the gastrointestinal tissues.

In the present application, the pathological or physiological process of any organ or tissue other than the gastrointestinal tract may comprise a systemic or local distal response caused by reperfusion to ischemic tissues in the gastrointestinal tract, and the response may comprise extensive microvascular dysfunction, a change in tissue barrier function and an inflammatory response.

In the present application, the pathological or physiological process of any organ or tissue other than the gastrointestinal tract may involve the lung.

For example, the pathological or physiological process of any organ or tissue other than the gastrointestinal tract may comprise pulmonary edema, an intrapulmonary thrombosis process, pulmonary embolism and/or inflammation of lung tissues.

In the present application, the pathological or physiological process of any organ or tissue other than the gastrointestinal tract may involve the kidney.

For example, the pathological or physiological process of any organ or tissue other than the gastrointestinal tract may comprise a renal failure, edematization, thrombosis, thromboembolism and/or inflammation of renal tissues.

In the present application, the pathological or physiological process of any organ or tissue other than the gastrointestinal tract may involve the central nervous system.

For example, the pathological or physiological process of any organ or tissue other than the gastrointestinal tract may comprise blood-brain barrier disruption, silent cerebral ischemia, cerebral stroke, cerebral edema, increased intracranial pressure, inflammation of neuronal tissues, nerve cell death, cerebral injury and/or nervous system dysfunction.

For example, the pathological or physiological process of any organ or tissue other than the gastrointestinal tract may comprise a cerebral ischemic stroke and related conditions.

For example, the cerebral ischemic stroke and related conditions may involve the following manifestations in a mouse MCAO model: unstained (grey) region of cerebral ischemic stroke in TTC staining, disappearance of purple Nissl bodies in the region of cerebral ischemic stroke, or an increase in the mouse modified neurological severity score.

For example, the pathological or physiological process of any organ or tissue other than the gastrointestinal tract may comprise an inflammation.

For example, the pathological or physiological process of any organ or tissue other than the gastrointestinal tract may comprise a systemic inflammation. The inflammation may involve the lung, gastrointestinal system, cardiovascular system, other extremities and/or central nervous system.

For example, the pathological or physiological process of any organ or tissue other than the gastrointestinal tract may comprise a systemic inflammatory response syndrome (intensive inflammatory response syndrome) and/or multiple organ dysfunction syndrome (MODS).

For example, the pathological or physiological process of any organ or tissue other than the gastrointestinal tract may comprise a disease, condition, or medical intervention inducing the gastrointestinal ischemia-reperfusion, for example, a cerebral stroke, a trauma, a shock, septicemia, acute pancreatitis, an inflammatory bowel disease, or a traumatic brain surgery.

For example, the trauma comprises damage to a human tissue or organ caused by an external factor, for example, traffic injury, injury from fall height, mechanical injury, sharp instrument injury, injury from falling, and firearm injury.

For example, the shock generally refers to a clinical syndrome involving a sharp decrease of effective circulating blood volume, body compensation loss, tissue ischemia and hypoxia, and neuro-humoral factor imbalance after strong attacks of pathogenic factors to a body. Its main features may comprise insufficient microcirculation perfusion in important organs and tissues, a metabolic disorder, and the dysfunction of various systems in the body, for example, a hypovolemic shock, a vasodilatory shock, a cardiogenic shock. The hypovolemic shock may comprise a hemorrhagic shock, a burn shock, and a traumatic shock; and the vasodilatory shock may comprise a septic shock, an allergic shock, and a neurogenic shock.

In the present application, the disease associated with the nervous system may comprise blood-brain barrier disruption, silent cerebral ischemia, stroke, cerebral edema, increased intracranial pressure, neuronal tissue inflammation, neuronal cell death, brain injury and/or nervous system dysfunction.

For example, the nervous system dysfunction may comprise dyskinesia and a homeostasis disorder. The homeostasis disorder may comprise an innervation-related disorder in the activity of an internal organ, body fluid or blood circulatory system.

In the present application, the condition associated with the nervous system may comprise a cerebral ischemic disease and its related conditions.

For example, the cerebral ischemic disease may comprise a group of diseases which cause brain tissue damage due to the interruption or reduction of blood flow to the brain caused by a vascular obstruction, lesion, trauma, etc.

For example, the cerebral ischemic disease may comprise cerebral ischemic stroke and a related condition thereof.

For example, the disease associated with the cerebral ischemic stroke may comprise cerebral infarction.

For example, the cerebral ischemic stroke may comprise atherosclerotic occlusion of a large artery, cerebral embolism (embolic infarction), non-embolic infarction of a small deep perforating artery (lacunar cerebral infarction), and ischemia in a watershed region due to distal arteriostenosis and decreased cerebral blood flow (hemodynamic stroke).

For example, the condition associated with the cerebral ischemic stroke may comprise cerebral nerve cell death, cerebral function impairment, changes in olfaction/gustation/auditory or visual sensation, dysphagia and asthenocoria to light, physical dyskinesia, aphasia, changes in breathing and heart rate, neuroregulation disorders of other tissues or organs, loss of consciousness, etc.

For example, the condition associated with cerebral ischemic stroke may comprise any index in the NIHSS. NIHSS refers to the National Institute of Health stroke scale, and the scoring method of NIHSS can be found in Williams LS, Yilmaz EY, Lopez-Yunez AM. Retrospective Assessment of Initial Stroke Severity with The NIH Stroke Scale. Stroke. 2000; 31:858-862.

For example, the cerebral ischemic stroke and related conditions may have the following manifestations in a mouse MCAO model: unstained (grey) region of cerebral ischemic stroke in TTC staining, disappearance of Nissl bodies in the region of cerebral ischemic stroke, or an increase in the mouse modified neurological severity score. Not wishing to be bound by any particular theory, the following examples are merely to set forth the use of the nitric oxide synthase pathway inhibitor in preparation of a drug, the use of nitric oxide synthase in screening of a drug, a pharmaceutical composition comprising the nitric oxide synthase pathway inhibitor, etc., according to the present application, and are not intended to limit the scope of the present application.

### Examples

### Materials and reagents

SPF male C57BL/6 mice, aged 8-10 weeks and weighing 22-24 g, were provided by Guangdong Medical Laboratory Animal Center, with certificates of quality for laboratory animals. Stool sample genomic DNA extraction kits (MinkaGene Stool DNA Kit) are products from Minka Gene; primers are synthesized by Thermo Fisher; TaqMan reverse transcription reagents and SYBR Green are products from Takara Bio; ViiA 7 real-time PCR system is a product from Applied Biosystems; nitrate/nitrite assay kits are products from Sigma; TTC powder is a product of Sigma; D-Lac, LBP, and LPS serum ELISA kits are products from Elisa Lab; and other materials, reagents, etc., are commercially available, unless otherwise specified.

### Statistical analysis

R analysis software is used for analyzing non-microbiological informatics data. Normal distribution data are expressed as mean ± standard deviation, and non-normal distribution data are exhibited in the form of median (interquartile range). Kruskal-Wallis rank sum test or Mann-Whitney U test is used for a non-parametric test, and unpaired Student's test or ONE-WAY ANOVA is used for analysis in a parametric test. Categorical variables are expressed in proportions. Shapiro-Wilk test is used to check the normality of data. For microbiome analysis, the Adonis test implemented in QIIME 1.9.1 is used. P <0.05 (two-tailed) is considered a significant difference.

### Example 1. Grouping of laboratory mice

8-week-old SPF male C57BL/6 wild-type mice were selected as experimental subjects and randomly divided into the following groups: a SHAM group for sham operation treatment; an MCAO group for MCAO modeling treatment; a Post-AG group (AG group) ), prepared by performing AG intervention (100 mg/kg, gavage, 200 µl/time) on the mice after MCAO modeling; and a Pre-AG group, prepared by performing AG intervention (1 mg/ml, 3-5 µl of drinking water daily, for 5 days) on the mice before MCAO modeling.

### Example 2. Establishment of mouse middle cerebral artery occlusion (MCAO) model

The mice were weighed and anesthetized with tribromoethanol at 0.2 ml/10 g. A median incision of about 1 cm was cut in the neck, and the tissues were carefully removed to expose the right carotid triangle. The right common carotid artery, external carotid artery (which needs to be removed as high as possible until reaching the anterior cranial bifurcation) and internal carotid artery were carefully isolated; and the common carotid artery was ligated at its proximal end (with a slip knot), and the external carotid artery was coagulated at its subbranches by using an electrocoagulator, and ligated at its proximal end (with a slip knot) and distal end (with a dead knot). The external carotid artery was cut open; an occlusion suture (with an appropriate type selected according to the weight of the mouse) was inserted to the direction of the internal carotid artery, the insertion was stopped when a slight resistance was felt (approximately when a mark (1 cm from the head) of the occlusion suture was at the bifurcation of the common carotid artery), and the occlusion suture was immobilized; and the skin was then sutured. The occlusion suture was removed 1 hour after the occlusion suture was inserted, which was taken as the starting point of timing after stroke (i.e., the zero point after stroke). During the operation, the core body temperature of the mouse was kept at 37+0.5°C with a constant temperature blanket, until the mouse woke up after the anesthesia effect disappeared. This procedure was for the purpose of preventing hypothermia cerebral protection from affecting the establishment of a mouse cerebral ischemic model. During the surgical procedures, mice with excessive hemorrhage or with the modeling time exceeding 15 minutes were discarded and not comprised in the later experimental analysis study.

### Example 3. Observation of cecal blood flow in mice

The blood vessels in the cecum of the mice in the SHAM group and the MCAO group were observed in blood flow. Taking the times before the modeling operation, after insertion of the occlusion suture, after removal of the occlusion suture, and 1 hour after removal of the occlusion suture for the mice in the MCAO group as time points, the mice in the SHAM group were subjected to a sham operation, and the blood flow was observed at the corresponding time points. The specific operation procedures were as follows:
(1) before the operation, the mouse was anesthetized and were sterilized at the abdomen, the skin and muscle layer were incised layer by layer along the midline of abdomen to expose the cecum; (2) the cecal blood flow of the mouse was observed by using a laser speckle imaging system (RWS RFLSI Pro), wherein the panoramic imaging of the mouse cecum was first observed, a picture was taken, and then the dynamic images of the cecal blood flow were recorded for 10 min by software of the laser speckle imaging system (RWD RFLSI Pro), with an interval per frame being 1 s;
(2) the blood flow changes in a region of interest (ROI) were used for analysis, with the region of interest covering six vascular branches of the cecum, and after the recording, the values from the region of interest were extracted as blood flow values; and
(3) a relative regional blood flow value for different time points in a detection process was obtained by dividing an average blood flow value in the region of interest at a corresponding time by a blood flow value at an initial time point.

The core body temperature of each mouse was controlled at 37+0.5°C throughout the surgical procedures.

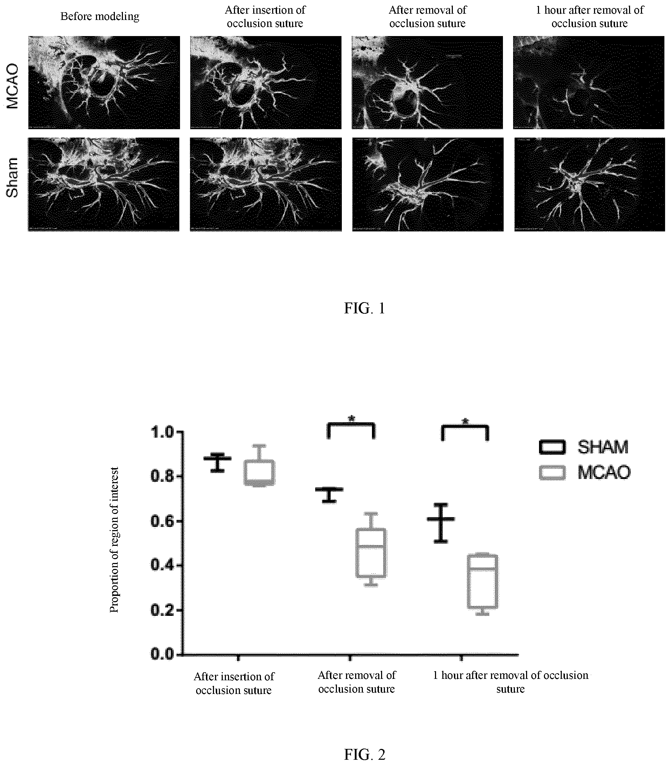

Observation results in FIG. 1 and FIG. 2 show that, at the time point of removal of the occlusion suture and 1 hour after this in the MCAO group, the cecal blood flow decreased in MCAO mice compared to that in SHAM mice, indicating that ischemia-reperfusion occurs in the intestines of the mice during cerebral stroke in the MCAO mice.

### Example 4. Quantitative analysis of expression levels of Nos2, Nox1, and Duox2 genes in intestinal tissues

At different time points (3 hours, 6 hours, 12 hours, 24 hours, 3 days, and 7 days, where the start time of timing is the time at which the occlusion suture was removed) after the cerebral stroke, the colon tissues of mice were rapidly collected and cryopreserved at -80°C for determining the relative expression level of *Nos2* gene. The specific steps were as follows.
(1) the colon tissues were homogenized by using a Minibeadbeater (Biospec Products, Bartlesville);
(2) RNA extraction was performed by a Trizol reagent method (Invitrogen): adding 1 ml of TRIZOL per 50-100 mg of homogenized tissue sample for homogenization; then, 0.2 ml of chloroform was added, and the sample was covered and shook vigorously for 15 seconds, held at 15-30°C for 2-3 min, and then centrifuged at 12,000 g for 15 min at 2-8°C; an upper aqueous phase was transferred, 0.5 ml of isopropanol was added, and the sample was held for 10 min at 15-30°C, and then centrifuged at 12,000 g for 10 min at 2-8°C; 1 ml of 75% ethanol was added to wash RNA pellets for 5 min; then, the RNA was dissolved to measure the concentration;
(3) cDNA was obtained from each RNA sample separately by using a TaqMan reverse transcription reagent (Takara Bio);
(4) 2 µl of cDNA added in 20 µl volume was taken as a template (at a concentration of 50-100 ng/µl), and upstream and downstream primers as set forth in SEQ ID NOs: 1-2 and 23-28 were added at a final concentration of 250 nM to carry out SYBR Green (Takara Bio) real-time PCR, with a reaction system shown in Table 1:

**Table 1**

| Reactant | Volume |
|---|---|
| Upstream primer F | 0.4µl |
| Downstream primer R | 0.4µl |
| ROX Reference Dye II | 0.4µl |
| SYBR Green | 10µl |
| cDNA | 2µl |
| PCR water | 6.8µl |

(5) data were extracted with the ViiA 7 real-time PCR system (Applied Biosystems), and data analysis was performed by using the comparative Ct method. The respective *Gapdh* expression levels were taken as internal references.

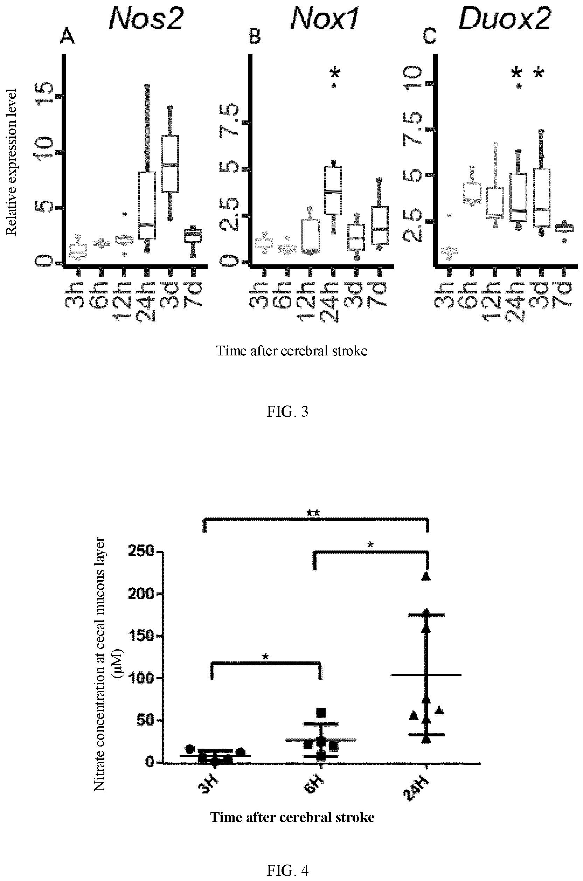

The results in FIGs. 3A-3C show that the expressions of *Nos2, Nox1*, and *Duox2* genes begin to increase within 3-6 hours after cerebral stroke, peaked at 12-24 hours.

### Example 5. Determination of nitrate concentration in cecal mucus extract

The nitrate concentration in the cecal mucus extract was measured at three time points, namely, 3 hours, 6 hours, and 24 hours after cerebral stroke. The specific steps were as follows:
(1) the contents in the cecal lumen of the mouse were removed, and the cecal mucus layer was collected and weighed;
(2) the cecal mucus of each mouse was extracted with 0.2 ml of ultrapure water (Gibco), and centrifuged for 2 min at 4°C and 20,000 g to remove larger particles, and the supernatant was filtered, sterilized, and stored at -80°C for later use;
(3) the sample obtained in step (2) was taken to make a standard curve following the steps in the instructions of the nitrate/nitrite detection kit (colorimetry) (Sigma), and the nitrate concentration was calculated according to the standard curve.

The results in FIG. 4 show that the nitrate concentration in the cecal mucous layer of the mouse significantly increases from 3 hours to 6 hours and then to 24 hours after the cerebral stroke.

### Example 6. Effect of aminoguanidine (AG) intervention on the expression level of Nos2 gene in intestinal tissues

The following three groups of mice were prepared as described in Example 1: a SHAM group treated with a sham operation; an MCAO group treated with MCAO modeling; a Post-AG group, prepared by performing AG intervention (100 mg/kg, 0.2 ml, gavage, once) 1 hour after cerebral stroke (i.e., 1 hour after removal of the occlusion suture) after the mice were subjected to MCAO modeling, which was carried out according to the method described in Example 2. 24 hours after cerebral stroke, the three groups of mice were separately sampled according to the method described in Example 4, and the expression level of Nos2 gene in intestinal tissues was determined.

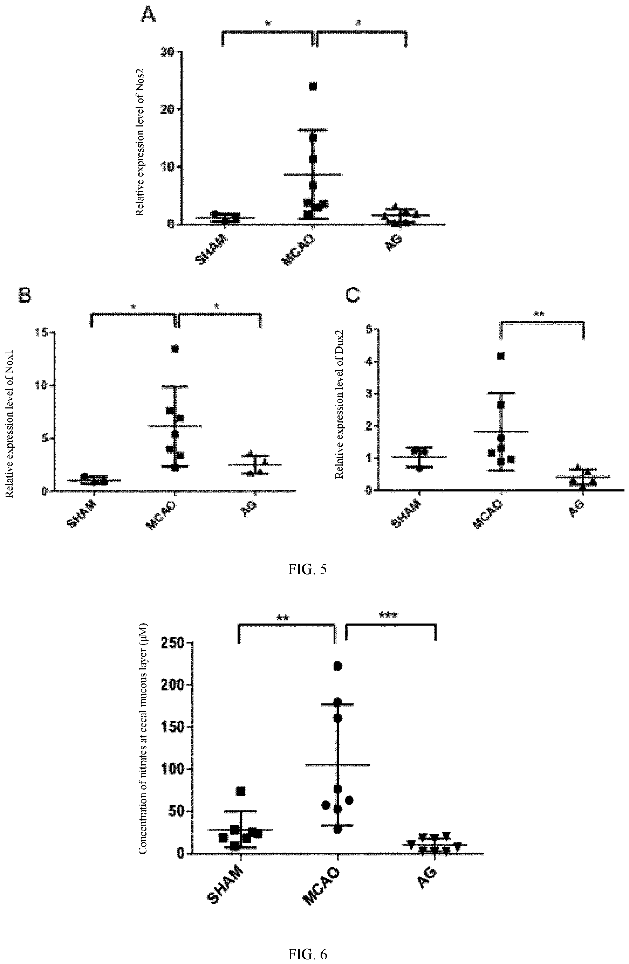

The results in FIG. 5A show that aminoguanidine (AG) significantly reduces the expression level of *Nos2* gene in the intestinal tissues of mice after cerebral stroke.

### Example 7. Effect of aminoguanidine (AG) intervention on the expression levels of Nox1 and Duox2 genes in intestinal tissues

The following three groups of mice were prepared as described in Example 1: a SHAM group treated with a sham operation; an MCAO group treated with MCAO modeling; a Post-AG group, prepared by performing AG intervention (100 mg/kg, 0.2 ml, gavage, once) 1 hour after cerebral stroke (i.e., 1 hour after removal of the occlusion suture) after the mice were subjected to MCAO modeling, which was carried out according to the method described in Example 2. 24 hours after cerebral stroke, the three groups of mice were separately sampled according to the method described in Example 4, and the expression levels of *Nox1* and *Duox2* genes in intestinal tissues were determined.

The results in FIGs. 5B-5C show that aminoguanidine (AG) significantly reduces the expression levels of *Nox1* 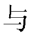 *Duox2* genes in the intestinal tissues of mice after cerebral stroke.

### Example 8. Effect of aminoguanidine (AG) intervention on nitrate concentration in cecal mucus extract

The following three groups of mice were prepared as described in Example 1: a SHAM group; an MCAO group treated with MCAO modelingt; a Post-AG group, prepared by performing AG intervention (100 mg/kg, gavage, once) 1 hour after cerebral stroke (i.e., 1 hour after removal of the occlusion suture) after the mice were subjected to MCAO modeling, which was carried out according to the method described in Example 2. 24 hours after cerebral stroke, the three groups of mice were separately sampled according to the method described in Example 5, and the nitrate concentration in the cecal mucus extract from the mice was determined.

The results in FIG. 6 show that aminoguanidine (AG) significantly reduces the nitrate concentration in the cecal mucus extract from the mice after cerebral stroke.

### Example 9. Effect of aminoguanidine (AG) intervention on the expression level of intestinal barrier factors and proinflammatory cytokines in intestinal tissues

The expression levels of *Tjp1*, *Ocln, Cldn2, Tnf, Il17, Ifng, Il1b, Cxcl2, Kc, and Il6* were detected according to the method of Example 4. *Tjp1*, *Ocln,* and *Cldn2* were intestinal barrier-related genes; *Tjp1* was a tight junction protein 1; *Ocln* was Occludin; *Cldn2* was Claudin-2; Tnf was a tumor necrosis factor; 1117 was interleukin 17; Ifng interferon γ; Il1b was Interleukin 1β; Il6 was interleukin 6; and Kc and Cxcl2 were chemokines. Samples were taken 24 hours after cerebral stroke. The primer sequences for *Tjp1*, *Ocln, Cldn2, Tnf, Il17*, *Ifng, Il1b, Cxcl2, Kc,* and *Il6* were as set forth in SEQ ID NOs: 3-22.

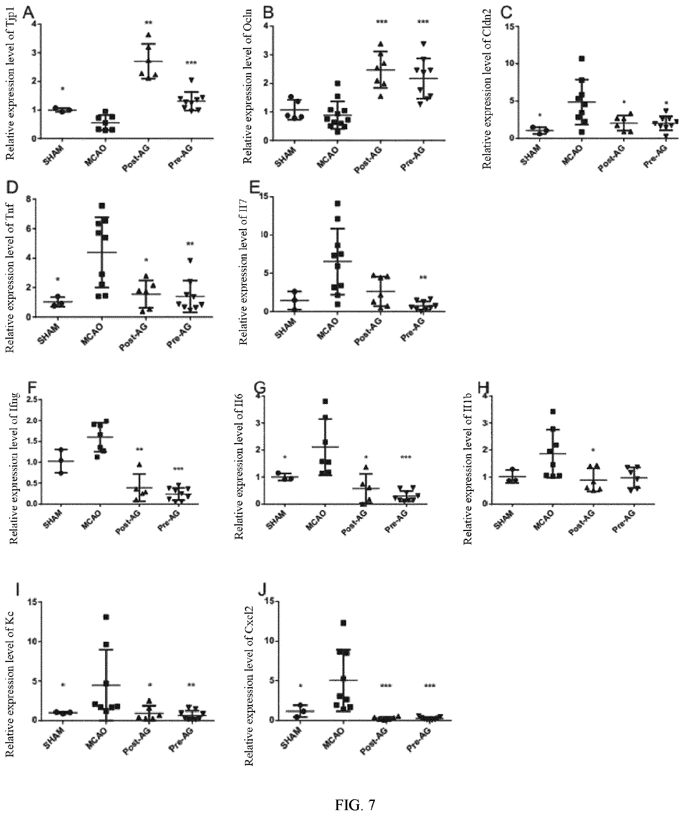

The results in FIGs. 7A-7J show that aminoguanidine (AG) intervention before or after modeling significantly increase the expression levels of *Tjp1* and *Ocln* genes, while the expression level of *Cldn2* gene significantly decreases, indicating that the intestinal barrier injury is alleviated; and at the same time, the expression levels of *Tnf, Il17, Ifng, Il1b, Cxcl2, Kc, and Il6* significantly decrease, indicating that the aminoguanidine (AG) intervention effectively inhibits the inflammatory response of colon tissues.

### Example 10. Effect of aminoguanidine (AG) intervention on brain injury in cerebral ischemic stroke

(1) The following three groups of mice were prepared as described in Example 1: an MCAO group treated with MCAO modeling; a Post-AG group, prepared by performing AG intervention (100 mg/kg, gavage, once) 1 hour after cerebral stroke (i.e., 1 hour after removal of the occlusion suture) after the mice were subjected to MCAO modeling; and a Post-AG group, prepared by performing AG intervention (1 mg/ml, 3-5 µl of drinking water daily, for 5 days) on mice before MCAO modeling, and performing MCAO modeling after 5 days of AG intervention; the MCAO modeling was carried out according to the method described in Example 2;
(2) TTC powder was prepared by using PBS, and a TTC staining solution had a concentration of 2%, and held in the dark;
(3) after the mouse was anesthetized (0.2 ml/10g of tribromoethanol), the thoracic cavity was opened, the right atrial appendage was cut open, and the heart was perfused with frozen PBS for about 2 min;
(4) the brain was removed by decapitation, the brain tissue was placed in a brain tank, which was then held in a refrigerator at -80°C for 7-8 min and then taken out, and the brain tissue was cut into coronal sections with a thickness of 1.5;
(5) the cut brain tissue was placed in the TTC staining solution and stained in the dark for 10 min, wherein a normal brain tissue was dark red, and an injury area with cerebral ischemic stroke was not stained (grey-white); formaldehyde was added to fix the stained sections, which were then taken out and photographed; and
(6) a cerebral injury volume was analyzed by using ImagePlus Soft image software. A cerebral edema factor was excluded during result analysis, the calculation was performed by the following formula of edema-corrected occlusion volume: cerebral injury area = direct injury volume - (somatic ipsilateral hemisphere - somatic contralateral hemisphere), and finally the proportion of cerebral injury volume in the whole cerebral hemisphere was obtained.

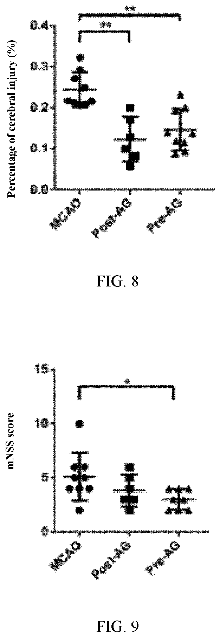

The results in FIG.8 show that regardless of the aminoguanidine (AG) intervention before or after modeling, the degree of injury of cerebral ischemic stroke in mice was significantly alleviated as compared with the MCAO group without intervention. This indicates that by inhibiting the expression of intestinal nitric oxide synthase (iNOS), the cerebral injury after cerebral ischemic stroke in mice can be improved.

### Example 11. Mouse modified neurological severity score (mNSS)

This operation was performed on the MCAO group, Post-AG group and Pre-AG group 24 hours after cerebral stroke. Neurobehavioral testing was performed by two testers blinded to the experimental grouping. The mNSS ranges from 0 to 14, with 0 representing normal and 14 representing the highest neurological severity. The scoring system can comprehensively assess neurological functions, comprising motor, sensory, balance and reflex tests. CD Motor test: the tail of the mouse was lifted to assess the degree of bending and twisting of the limbs (0-3 points); the walking posture on a flat surface was assessed (0-3 points). ②Balance test: the mouse was placed on a beam. Neurological impairment was mainly assessed based on whether the mouse could keep balance on the beam, dangle their limbs from the beam, and move smoothly through the beam (0-6 points). ③ Sensory and reflex test: auricle reflex and corneal reflex were tested respectively (0-2 points).

The results in FIG. 9 show that both the aminoguanidine (AG) interventions before and after modeling significantly improved the neurological deficit of mice.

### Example 12. Nissl staining of frozen sections of brain tissues

(1) Brain harvesting: the mice in each group (SHAM group, MCAO group, and Post-AG group) were sacrificed to harvest their brains after evaluating mNSS 24 hours after stroke.
(2) Immobilization: after the mice were anesthetized, the thoracic cavity was opened to fully expose the heart, the right atrial appendage was cut open, and the heart was perfused with frozen normal saline for 10 min, and then with frozen 4% paraformaldehyde (PFA) for 10 min. Tissues were then immobilized in PFA for 24 h, and in 30% PFA sucrose for 48 h. After sufficient dehydration, the brain tissues completely sunk to the bottom, and were taken out.
(3) Embedding: the brain tissues were excised and placed in a frozen section embedding mold, embedded in conventional OCT, frozen in liquid nitrogen, and stored in a refrigerator at -80°C.
(4) Sectioning: the temperature of a freezing microtome was adjusted to -20°C. After reaching the temperature, the specimens were taken out, and the brain tissues was taken from 2.0 mm to 4.0 mm behind the bregma (comprising the entire thalamus tissue), and successively cut into coronal sections with a thickness of 5 µm. The sections were flattened with a fine brush, placed on a glass slide, and stored in a refrigerator at -20°C.
(5) Nissl staining: the brain sections were immersed in the Nissl staining solution for 5 min and washed twice with distilled water. The sections were treated with 95% ethanol for about 5 seconds, and dehydrated in 95% ethanol for 2 min. The sections were immersed in fresh xylene for 5 min and mounted on slides with neutral gum. The slides were aired and then placed under a microscope for observation, and images were captured for analysis. The cell membranes of normal viable neurons were intact and blue-purple, and the nuclei were deeply stained.

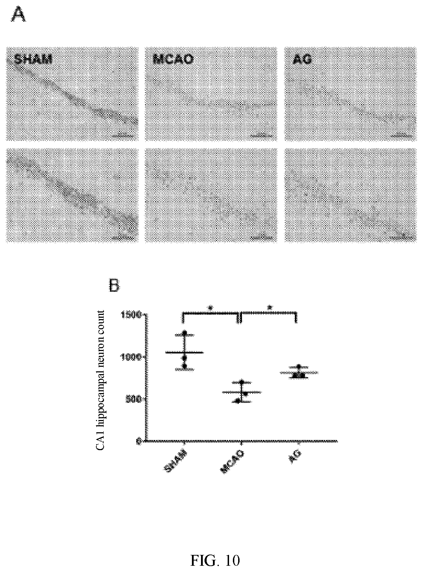

The results in FIGs. 10A-10B show that the AG intervention significantly reduce neuronal loss in the hippocampus regions of the mice.

### Example 13. Effect of aminoguanidine (AG) intervention on the levels of intestinal barrier markers and inflammatory factors in serum

After assessment of mNSS, blood collecting was performed on the mice. Blood collection methods comprised orbital blood collection and postcaval vein blood collection. Before testing, the mouse serum samples were stored at -80°C. Operations on the intestinal barrier markers such as D-Lac, LBP, and LPS in the mouse serum were performed following the steps in the instructions of the ELISA kit (Elisa Lab), and operations for the levels of inflammatory factors such as IL-6 and TNF-α were performed strictly following the steps in the instructions of the ELISA kit (Elisa Lab), whereby a standard curve was made, and respective expression levels were calculated according to the standard curve.

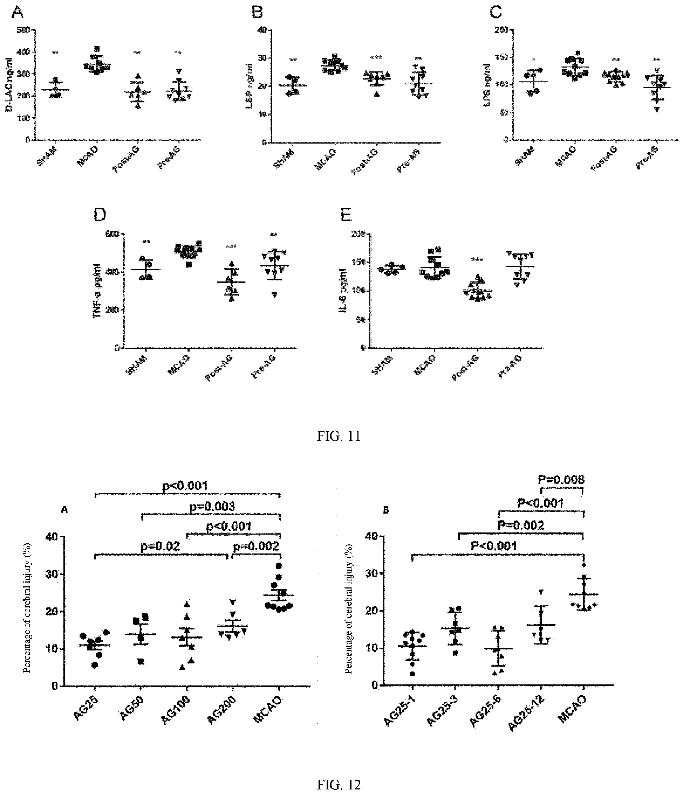

The results in FIGs. 11A-11E show that the aminoguanidine (AG) interventions before or after modeling significantly reduce the levels of D-Lac, LBP and LPS in the mouse serum, indicating that they improved the intestinal barrier injury; and at the same time, the aminoguanidine (AG) intervention after modeling significantly reduce the levels of IL-6 and TNF-α in the mouse serum, indicating that it effectively inhibits the inflammatory response.

### Example 14. Effects of aminoguanidine (AG) intervention at different times or doses on brain injury in cerebral ischemic stroke

The effects of aminoguanidine (AG) intervention at different times or doses on cerebral ischemic stroke after intestinal ischemia-reperfusion were tested according to the experimental method of Example 8.

Dose effect of AG: at the first hour after cerebral stroke in mice, the mice were treated with AG intervention at different doses, with a concentration range comprising: 25 mg/kg, 50 mg/kg, 100 mg/kg, and 200 mg/kg (200 µl by gavage, once). The degree of cerebral injury in mice was detected 24 hours after the cerebral stroke.

Time effect of AG: at the 1st, 3rd, 6th, and 12th hours after cerebral stroke in mice, the mice were treated with AG intervention (25 mg/kg, 200 µl by gavage, once). The degree of cerebral injury in mice was detected 24 hours after the cerebral stroke.

The results in FIGs. 12A-12B show that different doses of AG (range: 25-200 mg/kg) can significantly reduce cerebral injury in mice. In addition, the use of AG at different times after cerebral stroke (1-12 h after stroke) can also significantly reduce the degree of cerebral injury in mice.

### Example 15. Effect of 1400W, L-NIL or 2-amino-4-methylpyridine (AMP) intervention on the expression level of Nos2 gene in intestinal tissues

The following five groups of mice were prepared as described in Example 1: a SHAM group treated with a sham operation; an MCAO group treated with MCAO modeling; and a Post-1400W group/Post-L-NIL group/Post- AMP group, prepared by separately performing 1400W, L-NIL or AMP intervention (0.01-1000 mg/kg, 0.2 ml, gavage, once) 1 hour after cerebral stroke (i.e., 1 hour after removal of the occlusion suture) after the mice were subjected to MCAO modeling, which was carried out according to the method described in Example 2. 24 hours after cerebral stroke, the five groups of mice were separately sampled according to the method described in Example 4, and the expression level of *Nos2* gene in intestinal tissues was determined.

The results show that, compared to the MCAO group, the 1400W, L-NIL or 2-amino-4-methylpyridine (AMP) intervention reduces the expression level of *Nos2* gene in the intestinal tissues of mice after cerebral stroke.

### Example 16. Effect of 1400W, L-NIL, or 2-amino-4-methylpyridine (AMP) intervention on nitrate concentration in cecal mucus extract

The following five groups of mice were prepared as described in Example 1: a SHAM group treated with a sham operation; an MCAO group treated with MCAO modeling; and a Post-1400W group/Post-L-NIL group/Post- AMP group, prepared by separately performing 1400W, L-NIL or AMP intervention (0.01-1000 mg/kg, 0.2 ml, gavage, once) 1 hour after cerebral stroke (i.e., 1 hour after removal of the occlusion suture) after the mice were subjected to MCAO modeling, which was carried out according to the method described in Example 2. 24 hours after cerebral stroke, the five groups of mice were separately sampled according to the method described in Example 5, and the nitrate concentration in the cecal mucus extract was determined.

The results show that, compared to the MCAO group, the 1400W, L-NIL or 2-amino-4-methylpyridine (AMP) intervention reduces the nitrate concentration in the cecal mucus extract of mice after cerebral stroke.

### Example 17. Effect of 1400W, L-NIL, or 2-amino-4-methylpyridine (AMP) intervention on brain injury in cerebral ischemic stroke

The following four groups of mice were prepared as described in Example 1: an MCAO group treated with MCAO modeling; and a Post-1400W group/Post-L-NIL group/Post-AMP group, prepared by separately performing 1400W, L-NIL or AMP intervention (0.01-1000 mg/kg, gavage, once) 1 hour after cerebral stroke (i.e., 1 hour after removal of the occlusion suture) after the mice were subjected to MCAO modeling. The MCAO modeling was carried out according to the method described in Example 2. The effect of 1400W, L-NIL, or 2-amino-4-methylpyridine (AMP) intervention on the brain injury in cerebral ischemic stroke of mice was tested according to the method of Example 10.

The results show that, compared to the MCAO group without intervention, the 1400W, L-NIL or 2-amino-4-methylpyridine (AMP) intervention before or after modeling alleviates the degree of injury in the cerebral ischemic stroke of mice.

The foregoing detailed description is provided by way of explanation and examples, and is not intended to limit the scope of the appended claims. Various changes of the embodiments listed in the present application until now are obvious to those of ordinary skill in the art, and should be kept within the scope of the appended claims and equivalents thereof.

## Claims

1. Use of a nitric oxide synthase pathway inhibitor in preparation of a drug for preventing, alleviating and/or treating a distal injury associated with gastrointestinal ischemia-reperfusion in a subj ect.

2. The use according to claim 1, wherein said nitric oxide synthase pathway inhibitor inhibits the activity of nitric oxide synthase.

3. The use according to any one of claims 1-2, wherein said nitric oxide synthase comprises an inducible nitric oxide synthase iNOS.

4. The use according to any one of claims 1-3, wherein said nitric oxide synthase pathway inhibitor comprises an amino acid inhibitor and/or a non-amino acid inhibitor.

5. The use according to any one of claims 1-4, wherein said nitric oxide synthase pathway inhibitor comprises said amino acid inhibitor, which comprises aminoguanidine AG, 1400W, L-NIL and/or isothiourea.

6. The use according to any one of claims 1-4, wherein said nitric oxide synthase pathway inhibitor comprises said non-amino acid inhibitor, which comprises a glucocorticoid, a flavonoid, 2-amino-4-methylpyridine and/or aminopiperidine.

7. The use according to any one of claims 1-5, wherein said drug is formulated such that said nitric oxide synthase pathway inhibitor exerts an effect locally in a gastrointestinal tract.

8. The use according to any one of claims 1-7, wherein said nitric oxide synthase pathway inhibitor in said drug has a concentration of about 0. 0001% (w/w) to about 90% (w/w).

9. The use according to any one of claims 1-8, wherein said subject has experienced, is experiencing or is at risk of experiencing a surgical operation, disease or condition associated with said gastrointestinal ischemia-reperfusion.

10. The use according to claim 9, wherein said disease or condition associated with said gastrointestinal ischemia-reperfusion comprises a cerebral stroke, a trauma, a shock, septicemia, acute pancreatitis or an inflammatory bowel disease.

11. The use according to any one of claims 9-10, wherein said disease associated with said gastrointestinal ischemia-reperfusion comprises a cerebral ischemic stroke.

12. The use according to any one of claims 1-11, wherein said subject has experienced, is experiencing or is at risk of experiencing said gastrointestinal ischemia-reperfusion.

13. The use according to any one of claims 1-12, wherein said distal injury associated with said gastrointestinal ischemia-reperfusion comprises said cerebral ischemic stroke.

14. The use according to any one of claims 1-13, wherein said drug is formulated to adapt to oral administration.

15. The use according to any one of claims 1-14, wherein said nitric oxide synthase pathway inhibitor is not substantially decomposed and/or inactivated by digestive juices.

16. Use of a nitric oxide synthase for screening a drug for preventing, alleviating and/or treating a distal injury associated with gastrointestinal ischemia-reperfusion in a subject.

17. The use according to claim 16, wherein said drug inhibits the expression and/or activity of said nitric oxide synthase.

18. The use according to any one of claims 16-17, wherein said nitric oxide synthase comprises an inducible nitric oxide synthase iNOS.

19. A pharmaceutical composition comprising said nitric oxide synthase pathway inhibitor of any one of claims 1-15 and optionally a pharmaceutically acceptable carrier.

20. A method for preventing, alleviating and/or treating a distal injury associated with gastrointestinal ischemia-reperfusion in a subject, said method comprising administering to said subject said nitric oxide synthase pathway inhibitor of any one of claims 1-15.

21. The method according to claim 20, wherein said administering comprises gastrointestinal administration.

22. The method according to any one of claims 20-21, wherein said nitric oxide synthase pathway inhibitor exerts an effect locally in a gastrointestinal tract.

23. A method for preventing a distal injury associated with gastrointestinal ischemia-reperfusion in a subject, said method comprising:
(a) monitoring a gastrointestinal condition of said subject; and
(b) when said monitoring shows that said subject is at risk of experiencing said gastrointestinal ischemia-reperfusion, or during or after experiencing of said gastrointestinal ischemia-reperfusion, administering to said subject a nitric oxide synthase pathway inhibitor.

24. The method according to claim 23, wherein said administering comprises gastrointestinal administration.


Sequence Listing

<110>  Zhujiang Hospital, Southern Medical University

<120>  USE OF NITRIC OXIDE SYNTHASE PATHWAY INHIBITOR IN PERPARATION OF
MEDICINE

<130>  0133-PA-005EP

<150>  CN2019112746631
<151>  2019-12-12

<160>  28

<170>  PatentIn version 3.5

<210>  1
<211>  23
<212>  DNA
<213>  Artificial Sequence
<220>
<223>  gapdh upstream primer sequence

<400>  1
tgtagaccat gtagttgagg tca                                              23


<210>  2
<211>  21
<212>  DNA
<213>  Artificial Sequence
<220>
<223>  gapdh downstream primer sequence

<400>  2
aggtcggtgt gaacggattt g                                                21


<210>  3
<211>  20
<212>  DNA
<213>  Artificial Sequence
<220>
<223>  Ocln upstream primer sequence

<400>  3
acggaccctg accactatga                                                  20


<210>  4
<211>  20
<212>  DNA
<213>  Artificial Sequence
<220>
<223>  Ocln downstream primer sequence

<400>  4
tcagcagcag ccatgtactc                                                  20


<210>  5
<211>  20
<212>  DNA

<213>  Artificial Sequence
<220>
<223>  Cldn2 upstream primer sequence

<400>  5
tggttcctga cagcatgaaa                                                    20


<210>  6
<211>  20
<212>  DNA
<213>  Artificial Sequence
<220>
<223>  Cldn2 downstream primer sequence

<400>  6
ctttgggctg ttgagcagat                                                    20


<210>  7
<211>  22
<212>  DNA
<213>  Artificial Sequence
<220>
<223>  Tnf upstream primer sequence

<400>  7
ttgggtcttg ttcactccac gg                                                 22


<210>  8
<211>  24
<212>  DNA
<213>  Artificial Sequence
<220>
<223>  Tnf downstream primer sequence

<400>  8
cctctttcag gtcactttgg tagg                                               24


<210>  9
<211>  24
<212>  DNA
<213>  Artificial Sequence
<220>
<223>  Ifng upstream primer sequence

<400>  9
gctctgagac aatgaacgct acac                                               24


<210>  10
<211>  25
<212>  DNA
<213>  Artificial Sequence
<220>
<223>  Ifng downstream primer sequence

<400>  10
ttcttccaca tctatgccac ttgag                                              25

<210>  11
<211>  20
<212>  DNA
<213>  Artificial Sequence
<220>
<223>  Cxcl2 upstream primer sequence

<400>  11
tccaggtcag ttagccttgc                                                    20


<210>  12
<211>  20
<212>  DNA
<213>  Artificial Sequence
<220>
<223>  Cxcl2 downstream primer sequence

<400>  12
cggtcaaaaa gtttgccttg                                                    20


<210>  13
<211>  22
<212>  DNA
<213>  Artificial Sequence
<220>
<223>  Il6 upstream primer sequence

<400>  13
cggaggcttg gttacacatg tt                                                 22


<210>  14
<211>  23
<212>  DNA
<213>  Artificial Sequence
<220>
<223>  Il6 downstream primer sequence

<400>  14
ctggctttgt ctttcttgtt atc                                                23


<210>  15
<211>  20
<212>  DNA
<213>  Artificial Sequence
<220>
<223>  Kc upstream primer sequence

<400>  15
tgcacccaaa ccgaagtcat                                                    20


<210>  16
<211>  21
<212>  DNA
<213>  Artificial Sequence
<220>
<223>  Kc downstream primer sequence

<400>  16

ttgtcagaag ccagcgttca c                                              21

<210>  17
<211>  20
<212>  DNA
<213>  Artificial Sequence
<220>
<223>  Tjp1 upstream primer sequence

<400>  17
ggggcctaca ctgatcaaga                                                   20

<210>  18
<211>  20
<212>  DNA
<213>  Artificial Sequence
<220>
<223>  Tjp1 downstream primer sequence

<400>  18
tggagatgag gcttctgctt                                                   20

<210>  19
<211>  20
<212>  DNA
<213>  Artificial Sequence
<220>
<223>  Il-17 upstream primer sequence

<400>  19
acgtttctca gcaaacttac                                                   20

<210>  20
<211>  20
<212>  DNA
<213>  Artificial Sequence
<220>
<223>  Il-17 downstream primer sequence

<400>  20
cccctttaca ccttcttttc                                                   20

<210>  21
<211>  24
<212>  DNA
<213>  Artificial Sequence
<220>
<223>  Il1b upstream primer sequence

<400>  21
aagggctgct tccaaacctt tgac                                              24

<210>  22
<211>  24
<212>  DNA
<213>  Artificial Sequence
<220>

<223>  Il1b downstream primer sequence

<400>  22
tgcctgaagc tcttgttgat gtgc                                              24


<210>  23
<211>  21
<212>  DNA
<213>  Artificial Sequence
<220>
<223>  Nos2 upstream primer sequence

<400>  23
ccagccttgc atcctcattg g                                                 21


<210>  24
<211>  21
<212>  DNA
<213>  Artificial Sequence
<220>
<223>  Nos2 downstream primer sequence

<400>  24
ccaaacacca agctcatgcg g                                                 21


<210>  25
<211>  22
<212>  DNA
<213>  Artificial Sequence
<220>
<223>  Nox1 upstream primer sequence

<400>  25
gtgattacca aggttgtcat gc                                                22


<210>  26
<211>  20
<212>  DNA
<213>  Artificial Sequence
<220>
<223>  Nox1 downstream primer sequence

<400>  26
aagcctcgct tcctcatctg                                                   20


<210>  27
<211>  21
<212>  DNA
<213>  Artificial Sequence
<220>
<223>  Duox2 upstream primer sequence

<400>  27
acgcagctct gtgtcaaagg t                                                 21


<210>  28
<211>  21

<212> DNA
<213> Artificial Sequence
<220>
<223> Duox2 downstream primer sequence

<400> 28
tgatgaacga gactcgacag c 21